# EUROPEAN PATENT SPECIFICATION

(11) **EP 0 830 212 B1**
(45) Date of publication and mention of the grant of the patent: **27.08.2003**
(21) Application number: 96921360.2
(22) Date of filing: 06.06.1996
(51) Int. Cl.: B05B 5/025

(54) **APPARATUS FOR ELECTROSTATICALLY DEPOSITING AND RETAINING MATERIALS UPON A SUBSTRATE**
VORRICHTUNG ZUM ELEKTROSTATISCHEN AUFTRAGEN UND FESTHALTEN EINES MATERIALS AUF EIN SUBSTRAT
APPAREIL POUR LE DEPOT ELECTROSTATIQUE ET LA RETENUE DE MATIERES SUR UN SUBSTRAT

(30) Priority: 06.06.1995 US 467647
(43) Date of publication of application: 25.03.1998
(73) Proprietor: Delsys Pharmaceutical Corporation, Princeton, NJ 08540 (US)
(72) Inventor: PLETCHER, Timothy, Allen, Eastampton, NJ 08060 (US)
(74) Representative: Pratt, Richard Wilson
(86) International application number: PCT/US96/09439
(87) International publication number: WO 96/039256

(56) References cited:
- US-A- 2 408 143
- US-A- 3 854 043
- US-A- 4 197 289
- US-A- 5 014 076
- US-A- 5 286 918

## Description

The invention relates to electrostatic material deposition techniques and, more particularly to a substrate containing electrodes that provide electrostatic fields for retention of various materials upon the substrate.

US-A-2 408 143 proposes an apparatus for printing, which applies ink or other colouring fluids to the successive image areas only of a movable image carrying member by electrostatic or electronographic and/or magnetic lines of force created by one electrical circuit and then transferring the same from said successive image areas to correlated successive areas of the print receiving material by electrostatic or electronographic and/or magnetic lines of force created by a second and separate electric circuit.

The moveable image carrying member is electrically insulated from both electrical circuits and is provided with image areas that are permeable to lines of force and non-image areas which are impermeable thereto. This may be accomplished by coating the non-image areas with suitable material that is impermeable to lines of force while leaving the image areas free of said coating material. Then the ink or other colouring fluid is transferred by the lines of force created by the first electrical circuit from an ink discharging element to the image areas only of the image carrying member. The ink discharging element cooperates with an ink attraction element electrically connected in circuit with it but separated from the discharge element by the movable image carrying member. The ink or other colouring fluid which is deposited on the image areas only of the movable image carrying member is then transferred from said areas to correlated successive areas of the print receiving material by lines of force created by the second and separate electric circuit. The print receiving material passes between the image carrying member and supporting member and is supported by the latter. A repulsion element or blade is operatively associated with the image carrying member and an attraction element or blade is operatively associated with the supporting member, with said elements or blades electrically connected in the second and separate electric circuit but separated by the supporting member, the print receiving material and the image carrying member, wherefore lines of force passing across the gap between said blades pass through both members and the print receiving material. The supporting member may also have operatively associated therewith magnets for creating a magnetic field of force extending through both members and the print receiving material and acting to transfer the ink or colouring fluid from the image areas to the successive areas of said material.

Electrostatic deposition of materials such as toner powders is typically accomplished using an ion gun or print head to deposit a charge pattern upon a dielectric substrate. In operation, the print head scans a dielectric substrate and selectively deposits on the substrate a pattern of charge. The charge pattern is then exposed to a cloud of oppositely charged powder particles and the charge pattern attracts the powder to the substrate. The powder adheres to the substrate via electrostatic forces between the charged substrate and the oppositely charged powder. If the powder, for example, is a printing toner, then the substrate (e.g., paper) is developed using heat to melt the toner powder such that the print pattern permanently adheres to the substrate. As discussed above, such prior art material deposition systems use a print head that is separate from the substrate. The systems mechanically scan the head over the substrate to produce an accurate charge pattern. Such mechanical scanning requires a complicated head scanning mechanism. Such a mechanism is generally required where the pattern is constantly changing from one printing job to the next, e.g., systems that print text or graphics. However, other printing applications require repeated deposition of a particular charge quantity in a predefined pattern. Such an application includes retention of powdered drugs (medicament) at predefined locations on a substrate that is a component of a medication inhaler. Another such application is a printing stamp requiring a repeated pattern to be generated with each use of the stamp. Although in both of these applications, an identical charge pattern is repeatedly deposited on a substrate, the use of a prior art print head that robotically scans the substrate and deposits charge thereupon requires costly robotics.

Therefore, a need exists in the art for a material deposition technique that deposits and retains a deposition material directly upon the substrate without the use of a print head to generate a charge pattern.

### SUMMARY OF THE INVENTION

According to one aspect of the present invention, there is provided apparatus for electrostatically retaining a deposition material, said apparatus comprising:
a substrate including a dielectric layer, a conductive plate disposed on a first surface of said dielectric layer, and a collection trace, disposed on a second surface of said dielectric layer, where said conductive plate and said collection trace have a parallel spaced apart relation; wherein a voltage differential is established across the dielectric layer between the collection trace and the conductive place the collection trace having a charge of a first polarity; and
a deposition material, having a second charge polarity that is opposite the first charge polarity, said collection trace electrostatically attracting and retaining said deposition material.

According to a second aspect of the present invention, there is provided a method of electrostatically depositing a dose of medicament, comprising the steps of:
(a) charging a conductive surface, which is disposed in a spaced-apart parallel relation to a collection trace, to establish a voltage differential between the conductive surface and the collection trace;
(b) accumulating an electric charge at the collection trace, said charge being directly proportional to the voltage differential established in step (a);
(c) charging medicament particles to a polarity opposite that of the collection trace; and
(d) disposing the charged medicament particles proximate the collection trace, whereby the medicament is electrostatically attracted to the collection trace.

According to a third aspect of the present invention, there is provided apparatus for electrostatically retaining a deposition material, said apparatus comprising:
a substrate including a dielectric layer,
a conductive plate disposed on a first surface of said dielectric layer, and a collection trace disposed on a second surface of said dielectric layer, where said conductive plate and said collection trace have a parallel spaced apart relation; and
a voltage source selectively connectable to said conductive plate and said collection trace, for establishing a voltage differential across the dielectric layer between the collective trace and the conductive plate and charging said collection trace at a first charge polarity ;
whereby a deposition material, having a second charge polarity that is opposite the first charge polarity, may be electrostatically attracted and retained on said collection trace.

Specifically, an embodiment of the present invention provides a substrate having a planar conductive plating located on a first surface of a dielectric layer and having a conductive trace (a collection trace) located on a second surface of the dielectric layer such that the conductive plating and the conductive trace have a parallel, spaced-apart relation with respect to one another. The conductive trace is charged by supplying a voltage to both the plating and the trace to establish a voltage differential across the dielectric layer. As such, depending upon the magnitude of the voltage, polarity of the voltage and the duration for which the voltage is applied to the trace, a particular quantity and polarity of charge accumulates on the trace.

The material to be deposited is charged to an opposite polarity than that of the trace and then the deposition material is applied to the trace. Typically, if the deposition material is a powder, such as a powdered medicament, the powder is charged in a tribo-electric charging gun. If the material is a liquid, such as an ink, the liquid is charged using corona discharge apparatus within a liquid atomizer. In either case, the charged material is disposed over the charged collection trace and is electrostatically attracted to the trace. As such, the material adheres to the trace. The quantity of material adhered is directly proportional to the charge on the trace and the charge-to-mass ratio of the particles of deposition material. By determining the charge accumulated on the trace and the charge-to-mass ratio, a specific and repeatable quantity of deposition material is retained by the substrate.

The present invention provides a cost effective apparatus for repeatedly generating a well-defined charge pattern without using a robotically scanned print head. Applications for such apparatus include a substrate for retaining dry powder drugs within a drug inhaler and a substrate for retaining ink or toner in a programmable printing stamp.

### BRIEF DESCRIPTION OF THE DRAWINGS

The teachings of the present invention can be readily understood by considering the following detailed description in conjunction with the accompanying drawings, in which:
FIG. 1 depicts a perspective view of a substrate in accordance with the present invention;
FIG. 2 depicts a cross-sectional view of the substrate of FIG. 1 taken along line 2-2;
FIG. 3 depicts an illustrative application for the present invention; namely, a powdered medicament inhaler;
FIG. 4 depicts a substrate for use in the inhaler of FIG. 3;
FIG. 5 depicts a cross-sectional view of an element in the substrate of FIG. 4 taken along line 5-5; and
FIG. 6 depicts a second illustrative application for the present invention; namely, a programmable dot-matrix printing stamp.

To facilitate understanding, identical reference numerals have been used, where possible, to designate identical elements that are common to the figures.

### DETAILED DESCRIPTION

The present invention is apparatus for electrostatically retaining a deposition material upon a substrate. In general, the apparatus contains a conductive plate, a dielectric layer located upon the plate, and a conductive trace located upon the dielectric layer in a parallel spaced-apart relation with respect to the conductive plate. In use, a voltage is temporarily applied between the plate and the trace to charge the trace. Thereafter, deposition material, having a charge opposite that of the charge on the trace, is disposed upon the charged trace. The charged deposition material is attracted to the charged trace and is electrostatically adhered thereto. As such, the apparatus utilizes a relatively simple technique for electrostatically retaining the deposition material without using a mechanically scanned print head to form a charge pattern in the substrate.

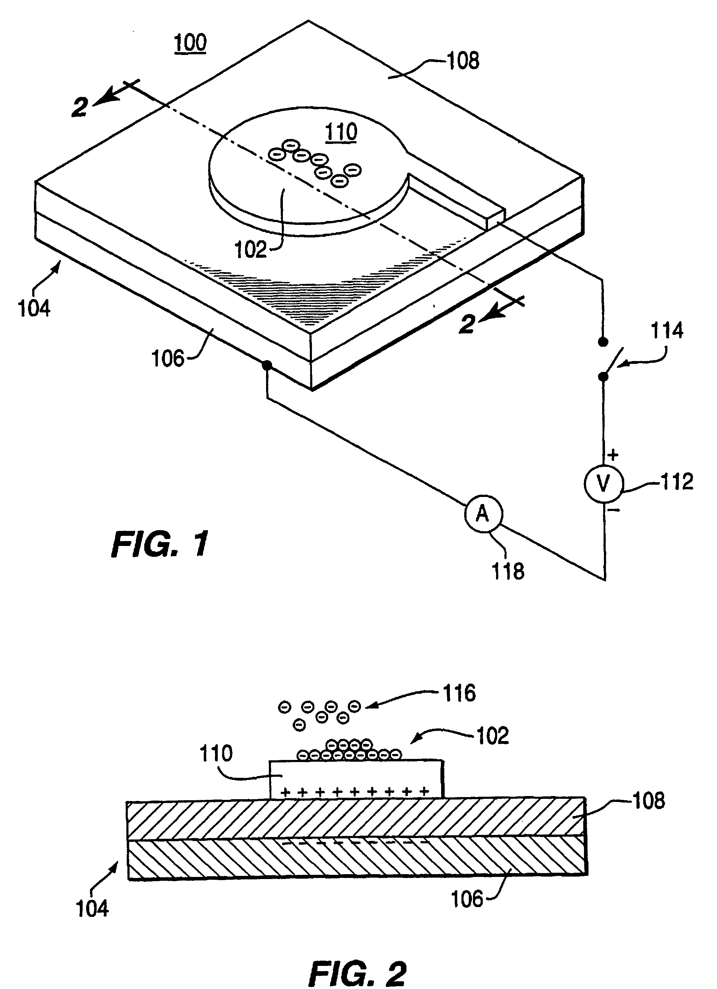

More specifically, FIG. 1 depicts a perspective view of the invention, while FIG. 2 depicts a cross-sectional view of a substrate 104 along line 2-2 of FIG. 1. To best understand the invention, the reader should consult both FIG. 1 and 2 while reading the following detailed description of the invention.

Apparatus 100 is designed to electrostatically retain a predefined quantity of deposition material 102 within a well-defined area upon a substrate 104. The apparatus contains the substrate 104, a voltage source 112, an ammeter 118, and a switch 114. The substrate 104 contains a conductive plate 106 and a conducive trace 110 (also referred to herein as a collection trace) separated from said conductive plate by a dielectric layer 108. The dielectric layer is typically alumina of thickness 5 micrometers. The conductors are typically created using a conventional thin film deposition process.

The collection trace 110 and plate 106 have a parallel spaced-apart relation with respect to one another. One terminal of a DC voltage source 112 is connected, through switch 114, to trace 110. The other terminal of source 112 is connected through ammeter 118 to plate 106. By temporarily closing the switch 114, the voltage source charges the collection trace with respect to the plate. The amount of charge accumulated on the trace during a specific period of time is directly proportional to the voltage differential applied between the plate and trace, i.e., the larger the differential, the greater the accumulated charge during a given time period. Typically, the current magnitude indicated by the ammeter 118 is monitored to determine a quantity of charge accumulated on the plate during a specified period of time that the switch is closed.

Illustratively, the trace is connected to the positive terminal of the source and the negative terminal of the source (ground) is connected to the plate. As such, upon closure of the switch, the trace is charged positively with respect to the plate. Of course, the plate could be charged negatively with respect to the plate without detrimentally affecting the operation of the inventive apparatus. The voltage used to charge the trace is a relatively low voltage, i.e., the voltage is typically on the order of tens to hundreds of volts. Given a dielectric thickness of 5 micrometers and a collection trace having a diameter of 1·02 mm (.040 inches). the capacitance between the trace and the plate is approximately 5 pF. With such a capacitance, the time required to charge the plate is approximately 50 picoseconds. To more easily monitor the charging process, the charge duration can be increased by adding a series resistor between the plate and the voltage source.

Once a particular charge is accumulated on the trace, a deposition material 116 is disposed over the charged collection trace. The material 116 is either a powder, such as a medicament or a printing toner, or a liquid, such as a printing ink. Whether a powder or a liquid is used, the material is electrically charged before being disposed over the trace. To facilitate electrostatic attraction between the material and the trace, the charge on the material is opposite the charge on the trace.

Typically, a powder deposition material is charged using a conventional tribo-electric charging technique (e.g., using what is commonly known as a tribo-electric charging gun) that provides a substantially uniform charge-to-mass ratio on the powder particles. The powder is charged at an opposite polarity to the charge on the trace. Once charged, the powder is expelled from the tribo-electric charging gun by a flow of air or some other gas. The expelled powder forms a cloud of charged powder particles proximate the charged collection trace. The trace attracts and retains the charged powder. Assuming a substantially uniform charge-to-mass ratio on the powder particles, the amount of powder that accumulates on the trace is directly proportional to the charge density on the trace. Furthermore, the dimensions of the trace define an area in which the powder is retained. Consequently, using the invention, a particular location on a substrate retains a particular amount of powder.

On the other hand, if the material to be deposited is a liquid, the liquid is typically charged by conventional corona charging apparatus within a liquid atomizer device. The liquid is typically charged as it is atomized within the atomizer. The atomizer expels the atomized liquid proximate to the charged collection trace. The trace attracts and retains the charged liquid. As such, the particular dimensions of the trace define an area in which the liquid is retained.

In some applications of the inventive substrate, it may be necessary to coat the substrate surface, including the collection trace, with a dielectric material. Such coating is typically required to ensure that the collection trace metal or the dielectric layer material of the substrate do not chemically react with the deposition material. A coating of this type should have little or no impact upon the operation and usefulness of the invention as described herein.

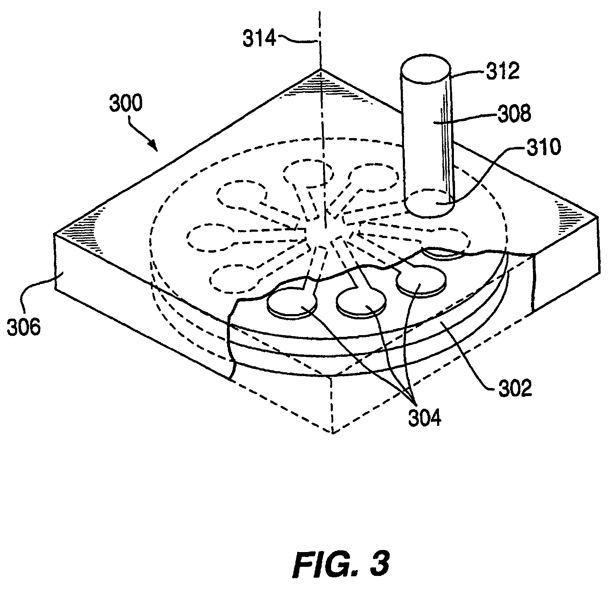

FIG. 3 depicts a specific application for the invention. In particular, the apparatus of FIG. 3 is a dry powder drug (medicament) inhaler 300 that is designed to retain a dry powdered drug at specific locations upon a substrate 302. As such, a powdered medicament is deposited in well defined doses in the manner discussed above and retained at particular locations upon the substrate until removed by an external force. The collection traces 304 that define the location and area upon which the medicament is retained typically have a diameter of 1·02 mm (.040 inches). In such an inhaler, a plurality of circular collection traces 304 are located on the substrate 302 to enable multiple doses of the medicament to be retained in well-defined locations upon a single substrate. A housing 306 encloses the substrate 302 and supports a flexible delivery tube 308. The substrate is rotatable about a central axis 314 with respect to the housing and the delivery tube. As such, the substrate can be rotated to align a particular medicament dose (i.e., a particular collection trace) with an inlet end 310 of the delivery tube 308. When a patient inhales through the outlet end 312 of the tube, the medicament is dislodged from the substrate and carried by air flow to the lungs of the patient. To promote sufficient air flow to dislodge the medicament, the substrate at each of the trace locations is perforated with a plurality of openings that are smaller than the smallest medicament particle. As such, air flows through the dose location, i.e., through perforations in the collection trace, the dielectric layer and the plate, to carry the medicament to the patient.

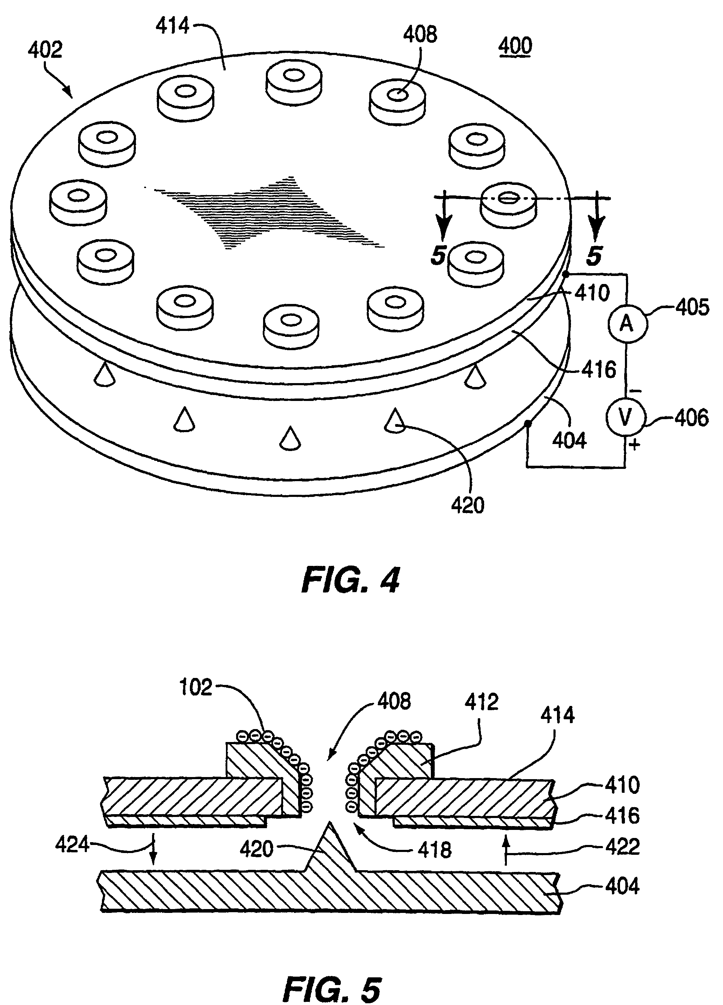

FIG. 4 depicts a perspective view of another embodiment of the inventive substrate for utilization in a dry powder medicament inhaler such as the inhaler of FIG. 3. FIG. 5 depicts a cross-sectional view of the substrate in FIG. 4 taken along line 5-5. To best understand this embodiment of the invention, the reader should consult both FIGS. 4 and 5 while reading the following detailed description.

Apparatus 400 contains substrate 402, charging plate 404, an ammeter 405, and voltage source 406. Each medicament dose location contains an opening 408 through a dielectric layer 410. The opening has deposited therein a collection trace 412 that plates the surface of the opening as well as an area on a top surface 414 of the dielectric layer 410. Underlying the entire dielectric layer 410 is a conductive plate 416. The plate contains apertures 418 that are coaxial with, but slightly larger in diameter than, the openings 408 through the dielectric layer 410. Furthermore, the apparatus contains a charging plate 404 fabricated of a conductive material and having a plurality of cone-shaped protuberances 420. The protuberances are aligned with the respective openings 408 and 418 in the dielectric layer 410 and the conductive plate 416. The protuberances are sized to enter the openings 408 in the dielectric layer and contact the trace plating 412 within each opening 408 without contacting the conductive plate 416. The charging plate is connected to one terminal of a DC voltage source 406 and the conductive plate is connected through an ammeter to the other terminal of the source. As such, when a protuberance of the charging plate contacts the collection trace, a charge accumulates on the trace. Such charging is accomplished by moving the charging plate upwards in a direction indicated by arrow 422. The charge accumulated is directly proportional to the voltage applied between the trace and the conductive plate. Once a particular charge is accumulated, the charging plate is removed from contact with the collection trace by moving the charging plate downwards in a direction indicated by arrow 424.

As with the previous embodiment, to retain a medicament powder, a cloud of charged medicament is generated proximate the collection traces. By having a medicament charge that is opposite that of the trace charge, the trace retains the medicament in a quantity that is proportional to the accumulated charge on the collection trace. Thus, the location and quantity (dose) of the medicament dose is strictly controlled.

Once medicament is deposited at all the locations on the substrate, the substrate 402 is used in an inhaler such as that described with respect to FIG. 3. In this embodiment, as a patient inhales through the delivery tube, air flow passes through the opening 408 in the dielectric layer 410 and dislodges the medicament from the trace 412. The airflow carries the dislodged medicament powder to the patient's lungs.

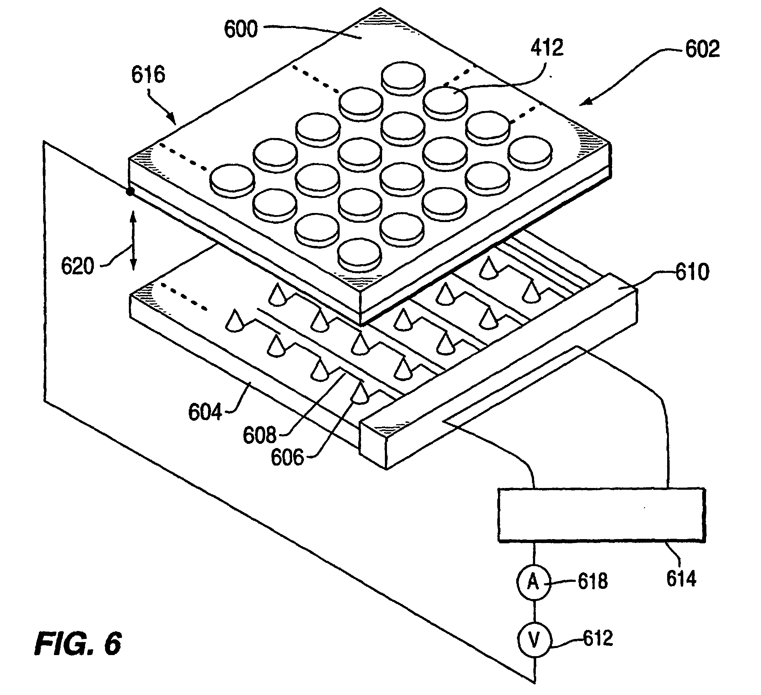

FIG. 6 depicts another alternative embodiment of the present invention that permits each collection trace to be selectively and individually charged. Specifically, the substrate 616 contains the collection traces 412 shown in FIG. 5 arranged in an array 602 upon a dielectric layer 600. The traces for this embodiment may or may not have an opening extending through the trace, i.e., the opening 408 may be filled with conductive material that comprises the collection trace 412. The charging plate 604, in the embodiment of FIG. 6, is fabricated of an insulator having a plurality of conductive protuberances 606 deposited upon its surface. The protuberances are connected to conductive traces 608 that lead to an edge connector 610 located at one edge of the charging plate. Each protuberance 606 is aligned with an associated collection trace 412 of the substrate 616.

In use, one terminal of a DC voltage source 612 is connected to the conductive plate 61 of the substrate, while the other terminal is connected, through an ammeter 618, to selected ones of the protuberances 606. Switching logic 614 such as programmable array logic (PAL), is used to select the protuberances that are to be connected to the voltage source. Once the selection is made, the charging plate is moved upwards along arrow 620 into contact with the substrate such that each of the protuberances 606 contact their associated collection trace 412. As such, the protuberances that are selectively connected to the voltage source 612 facilitate charging of their respective collection traces.

Once charged to a predetermined charge quantity as measured on the ammeter 618, a deposition material is deposited on the collection traces. As discussed above, a powder, such as a printing toner, may be charged and deposited using a tribo-electric charging technique, or a liquid, such as a printing ink, can be deposited using a corona charging technique in conjunction with a liquid atomizer. In either case, the charged deposition material adheres to only those collection traces that are charged. Thus, the material adheres in a pattern defined by the collection traces that are charged. Thereafter, the traces coated with deposition material can be used in various applications such as repetitive printing onto paper or other material.

For example, if the traces are selectively coated with ink to form a dot-matrix pattern, the traces can be pressed against paper, cloth, cardboard or some other material. Absorption of the ink into the material being printed removes the ink from the collection traces. As such, the substrate forms a programmable stamp that prints in dot-matrix patterns. To facilitate release of the ink, the charging plate can be reapplied to the collection traces while all the protuberances are grounded. In this manner, the electrostatic charge is removed from the collection traces such that the ink can easily be transferred to a surface, even a non-absorptive surface.

To change the printing pattern of the programmable stamp, a user merely needs to clear the previous pattern by grounding all the collection traces and then, by altering the switching logic, redefine which of the collection traces are to be charged. Once charged, the user may redeposit the ink. This same process also works for deposition of powdered toners. However, for printing with toners, one further process step is necessary. Once the toner is deposited on a material to be printed, that material is heated to fix the toner. Alternatively, rather than use a charging plate to charge the collection traces in any of the foregoing embodiments of the invention, the collection trace is charged using a conventional ion emitter or electron gun. As such, the emitter or gun is positioned proximate to the collection trace, activated, and, in response to bombardment by ions or electrons, the collection trace becomes charged.

Although various embodiments which incorporate the teachings of the present invention have been shown and described in detail herein, those skilled in the art can readily devise many other varied embodiments that still incorporate these teachings.

## Claims

1. Apparatus electrostatically retaining a deposition material, said apparatus comprising:
a substrate (104) including a dielectric layer (108, 410, 600), a conductive plate (106, 416, 617) disposed on a first surface of said dielectric layer, and a collection trace (110, 412) disposed on a second surface of said dielectric layer (108, 410, 600), where said conductive plate (106, 416, 617) and said collection trace (110, 412) have a parallel spaced apart relation; wherein a voltage differential is established across the dielectric layer (108) between the collection trace (110, 412) and the conductive plate (106, 416, 617), the collection trace having a charge of a first polarity; and
a deposition material (102), having a second charge polarity that is opposite the first charge polarity, said collection trace (110, 412) electrostatically attracting and retaining said deposition material (102).

2. The apparatus of claim 1, further comprising a voltage source (112, 406, 612) selectively connectable to said conductive plate (106, 416, 617) and said collection trace (110, 412), for establishing the said voltage differential and charging said collection trace (110, 412) with the first charge polarity.

3. The apparatus of claim 2, wherein said voltage source (112, 406, 612) is connected to said conductive plate (106, 416, 617) and said collection trace (110, 412) through an ammeter (118, 618).

4. The apparatus of claim 2 or 3, further comprising switch means (114; 404; 614, 604, 608, 606) for selectively connecting said voltage source (112, 612) to said collection trace (110, 412), for controlling the quantity of charge accumulated on said collection trace (110, 412).

5. The apparatus of claim 4, wherein said dielectric layer (410) defines an opening (408) and said collection trace (412) is disposed on said first surface proximate said opening and into said opening, and wherein said conductive plate (416) defines an opening (418) that is coaxially aligned with said opening (408) defined by said dielectric layer (410).

6. The apparatus of claim 5, wherein a first terminal of said voltage source (406) is connected to said conductive plate (416) and a second terminal of said voltage source (406) is connected to a charging plate (404) of the switch means.

7. The apparatus of claim 6, wherein said charging plate (404) comprises a protuberance (420) extending from a surface of said charging plate (404) and being formed to interfit said opening (418) in said dielectric layer (410) to contact said collection trace (412) without contacting said conductive plate (416), to charge the collection trace.

8. The apparatus of claim 7, wherein the charging plate is movable between a first position in which the protuberance contacts the collection trace and a second position in which the trace is not contacted.

9. The apparatus of any preceding claim, wherein said deposition material is a powder.

10. The apparatus of claim 9, wherein the deposition material is powdered medicament.

11. The apparatus of claim 10, further comprising means (308), positioned proximate the collection trace (412), for extracting said powdered medicament from said collection trace.

12. The apparatus of claim 11, wherein said extracting means (308) is a delivery tube providing for inhalation of said powdered medicament.

13. The apparatus of any one of claims 1 to 8, wherein said deposition material is a liquid.

14. Apparatus according to claim 1 wherein; the said substrate includes a dielectric layer (410) defining a plurality of openings (408); the said conductive plate (416) disposed on a first surface of said dielectric layer (410) defines openings coaxially aligned with respective openings (408) defined by said dielectric layer (410); and there are a plurality of collection traces (412), disposed on a second surface of said dielectric layer (410) proximate and into each of said openings (408) defined by said dielectric layer (410), the collection traces having a charge of a first polarity; and
each of said collection traces electrostatically attracts and retains said deposition material and having a second charge polarity that is opposite the first charge polarity.

15. The apparatus of claim 14, further comprising a voltage source (406) having a first terminal connected to said conductive plate (416) and a second terminal connected to a charging plate (404) for selectively charging each of said collection traces (412) with the first charge polarity.

16. The apparatus of claim 15, wherein said charging plate (404) comprises a plurality of protuberances (420) extending from a surface of said charging plate and being formed to interfit said openings (408) in said dielectric layer (410) to contact said collection traces (412) without contacting said conductive plate (416).

17. The apparatus of claim 15 or 16, wherein the charging plate is movable between a first position in which at least one of the protuberance contacts a collection trace and a second position in which none of the collection traces is contacted.

18. The apparatus of claim 14, 15, 16 or 17, further comprising:
means (306) supporting said substrate (410,416,412); and
means (308) for extracting said deposition material from the traces;
the traces (412) being positionable with respect to the extracting means (308) to allow the extracting means (308) to extract the material therefrom.

19. apparatus of claim 14, 15, 16, 17 or 18, wherein the deposition material is a medicament powder.

20. The apparatus of claim 19,wherein said extracting means is a delivery tube (308) providing for inhalation of said powdered medicament.

21. The apparatus of claim 13, 14, 15, 16 or 17, wherein each of said collection traces (412) fills each of said openings defined by said dielectric layer (600), the traces forming a dot matrix array of printing elements.

22. The apparatus of claim 21, further comprising switching logic (614), connected between said voltage source (612) and said charging plate (604), for selecting specific ones of said protuberances for connection to said voltage source.

23. The apparatus of claim 22, wherein said deposition material is printable substance.

24. A method of electrostatically depositing a dose of medicament, comprising the steps of:
(a) charging a conductive surface (416), which is disposed in a spaced-apart parallel relation to a collection trace (412), to establish a voltage differential between the conductive surface and the collection trace;
(b) accumulating an electric charge at the collection trace, said charge being directly proportional to the voltage differential established in step (a);
(c) charging medicament particles to a polarity opposite that of the collection trace; and
(d) disposing the charged medicament particles proximate the collection trace,
whereby the medicament is electrostatically attracted to the collection trace.

25. The method of claim 24, further comprising the step of determining the electric charge accumulated in step (b).

26. The method of claim 25, further comprising the step of discontinuing the charging of the conductive surface when a specified amount of electric charge has accumulated at the collection trace.

27. The method of claim 26, wherein the dose of medicament deposited on the collection trace is directly proportional to the electric charge accumulated at the collection trace.

28. The method of claims 24, 25, 26 or 27, wherein the collection trace defines a location in a predefined pattern where the medicament particles are to be deposited.

29. Apparatus for electrostatically retaining a deposition material, said apparatus comprising:
a substrate (104) including a dielectric layer (108, 410, 600),
a conductive plate (106, 416, 617) disposed on a first surface of said dielectric layer, and a collection trace (110, 412), disposed on a second surface of said dielectric layer (108, 410, 600), where said conductive plate (106, 416, 617) and said collection trace (110, 412) have a parallel spaced apart relation; and
a voltage source (112, 406, 612) selectively connectable to said conductive plate (106, 416, 617) and said collection trace (110, 412), for establishing a voltage differential across the dielectric layer (108) between the collective trace (110, 412) and the conductive plate (106, 416, 617 and charging said collection trace (110, 412) at a first charge polarity;
whereby a deposition material (102), having a second charge polarity that is opposite the first charge polarity, may be electrostatically attracted and retained on said collection trace (110, 412).

30. Apparatus according to claim 29, wherein:
the said substrate including a dielectric layer (410) defines a plurality of openings (408);
the said conductive plate (416), disposed on a first surface of said dielectric layer (410), defines openings coaxially aligned with respective openings (408) defined by said dielectric layer (410); and
there are plurality of collection traces (412), disposed on a second surface of said dielectric layer (410) proximate and into each of said openings (408) defined by said dielectric layer (410); and
the said voltage source (406) having a first terminal connected to said conductive plate (416) and a second terminal connected to a charging plate (404), is for selectively charging each of said collection traces (412) at a first charge polarity;
whereby a deposition material, having a second charge polarity that is opposite the first charge polarity may be electrostatically attracted and retained on said collection traces.

## Patentansprüche

1. Vorrichtung für das elektrostatische Aufnehmen eines Abscheidungsmaterials, wobei die Vorrichtung aufweist:
ein Substrat (104), das eine dielektrische Schicht (108, 410, 600), eine leitfähige Platte (106, 416, 617), die auf einer ersten Oberfläche der dielektrischen Schicht angeordnet ist, und eine Sammlungsspur (110, 412), die auf einer zweiten Oberfläche der dielektrischen Schicht (108, 410, 600) angeordnet ist, aufweist, wobei die leitfähige Platte (106, 416, 617) und die Sammlungsspur (110, 412) eine parallel zueinander beabstandete Beziehung aufweisen, wobei eine Spannungsdifferenz über der dielektrischen Schicht (108) zwischen der Sammlungsspur (110, 412) und der leiffähigen Platte (106, 416, 617) errichtet wird, wobei die Sammlungsspur eine Ladung einer ersten Polarität hat, und
ein Abscheidungsmaterial (102) mit einer zweiten Ladungspolarität, die entgegengesetzt zu der ersten Ladungspolarität ist, wobei die Ladungsspur (110, 412) das Abscheidungsmaterial (102) elektrostatisch anzieht und aufnimmt.

2. Vorrichtung nach Anspruch 1, die weiterhin eine Spannungsquelle (112, 406, 612) aufweist, die wahlweise mit der leitfähigen Platte (106, 416, 617) und der Sammlungsspur (110, 412) verbindbar ist für das Errichten der Spannungsdifferenz und das Laden der Sammlungsspur (110, 412) mit der ersten Ladungspolarität.

3. Vorrichtung nach Anspruch 2, wobei die Spannungsquelle (112, 406, 612) mit der leitfähigen Platte (106, 416, 617) und der Sammlungsspur (110, 412) über ein Amperemeter (118, 618) verbunden ist.

4. Vorrichtung nach Anspruch 2 oder 3, die weiterhin eine Schalteinrichtung (114; 404; 614, 604, 608, 606) für das wahlweise Verbinden der Spannungsquelle (112, 612) mit der Sammlungsspur (110, 412) aufweist für das Steuern der Ladungsmenge, die auf der Ladungsspur (110, 412) gesammelt wird.

5. Vorrichtung nach Anspruch 4, wobei die dielektrische Schicht (410) eine Öffnung (408) definiert und die Sammlungsspur (412) auf der ersten Oberfläche in der Nähe der besagten Öffnung und in der Öffnung angeordnet ist, und wobei die leitfähige Platte (416) eine Öffnung (418) definiert, die koaxial zu der Öffnung (408), die von der dielektrischen Schicht (410) gebildet wird, ausgerichtet ist.

6. Vorrichtung nach Anspruch 5, wobei ein erster Anschluß der Spannungsquelle (406) mit der leitfähigen Platte (416) verbunden ist und ein zweiter Anschluß der Spannungsquelle (406) mit einer Ladungsplatte (404) der Schalteinrichtung verbunden ist.

7. Vorrichtung nach Anspruch 6, wobei die Ladeplatte (404) eine Ausstülpung (420), die sich von einer Oberfläche der Ladeplatte (404) erstreckt und derart ausgebildet ist, daß sie die Öffnung (418) in der dielektrischen Schicht (410) zwischen verbindet, um die Sammlungsspur (412) zu kontaktieren, ohne die leitfähige Platte 8416) zu kontaktieren, aufweist, um die Sammlungsspur zu laden.

8. Vorrichtung nach Anspruch 7, wobei die Ladeplatte zwischen einer ersten Position, in der die Vorstülpung die Sammlungsspur kontaktiert, und einer zweiten Position, in der die Spur nicht kontaktiert wird, bewegbar ist.

9. Vorrichtung nach einem der vorherigen Ansprüche, wobei das Abscheidungsmaterial ein Pulver ist.

10. Vorrichtung nach Anspruch 9, wobei das Abscheidungsmaterial ein pulverförmiges Medikament ist.

11. Vorrichtung nach Anspruch 10, das weiterhin eine Einrichtung (308) aufweist, die in der Nähe der Sammlungsspur (412) angeordnet ist für das Extrahieren des gepulverten Medikaments von der Sammlungsspur.

12. Vorrichtung nach Anspruch 11, wobei die Extraktionseinrichtung (308) ein Zuführungsrohr ist, das die Inhalation des pulverförmigen Medikaments zur Verfügung stellt.

13. Vorrichtung nach einem der Ansprüche 1 bis 8, wobei das Abscheidungsmaterial eine Flüssigkeit ist.

14. Vorrichtung nach Anspruch 1, wobei das Substrat eine dielektrische Schicht (410), die eine Mehrzahl von Öffnungen (408) bildet, beinhaltet, die leitfähige Platte (416), die auf einer ersten Oberfläche der dielektrischen Schicht (410) angeordnet ist, Öffnungen definiert, die koaxial mit dem entsprechenden Öffnungen (408), die von der dielektrischen Schicht (410) gebildet werden, angeordnet sind und es eine Mehrzahl von Sammlungsspuren (412) gibt, die auf einer zweiten Oberfläche der dielektrischen Schicht (410) in der Nähe und in jeder der Öffnungen (408), die von der dielektrischen Schicht (410) gebildet werden, angeordnet sind, wobei die Sammlungsspuren eine Ladung einer ersten Polarität haben, und jede der Ladungsspuren elektrostatisch das Abscheidungsmaterial anzieht und aufnimmt und eine zweite Ladungspolarität hat, die entgegengesetzt zu der ersten Ladungspolarität ist.

15. Vorrichtung nach Anspruch 14, die weiterhin eine Spannungsquelle (406) mit einem ersten Anschlu8 hat, der mit der leitfähigen Platte (416) verbunden ist und einen zweiten Anschluß hat, der mit einer Ladeplatte (404) verbunden ist für das wahlweise Laden jeder der Sammlungsspuren (412) mit der ersten Ladungspolarität.

16. Vorrichtung nach Anspruch 15, wobei die Ladeplatte (404) eine Mehrzahl von Vorstülpungen (420) aufweist, die sich von einer Oberfläche der Ladungsplatte erstrecken und derart gebildet sind, daß sie um die Öffnungen (408) in der dielektrischen Schicht (410) passen, um die Ladungsspuren (412) zu kontaktieren, ohne einen Kontakt zu der leitfähigen Platte (416) herzustellen.

17. Vorrichtung nach Anspruch 15 oder 16, wobei die Ladungsplatte zwischen einer ersten Position, in der zumindest eine der Vorstülpungen eine Sammlungsspur kontaktiert, und einer zweiten Position, in der keine der Sammlungsspuren kontaktiert wird, bewegbar ist.

18. Vorrichtung nach Anspruch 14, 15, 16 oder 17, die weiterhin aufweist:
eine Einrichtung (306), die das Substrat (410, 416, 412) trägt, und
eine Einrichtung (308) für das Extrahieren des Abscheidungsmaterials von den Spuren,
wobei die Spuren (412) in Bezug auf die Extrahierungseinrichtung (308) positionierbar s ind, um es der Extrahierungseinrichtung (308) zu erlauben, das Material hiervon zu extrahieren.

19. Vorrichtung nach Anspruch 14, 15, 16, 17 oder 18, wobei das Abscheidungsmaterial ein Medikamentenpulver ist.

20. Vorrichtung nach Anspruch 19, wobei die Extrahierungseinrichtung ein Zuführungsrohr (308) ist, das die Inhalation des pulverförmigen Medikaments zur Verfügung stellt.

21. Vorrichtung nach Anspruch 13, 14, 15, 16 oder 17, wobei jede der Sammlungsspuren (412) jeder der Öffnung, die von der dielektrischen Schicht (600) gebildet werden, füllt, wobei die Spuren eine Punktmatrixanordnung von Druckelementen bilden.

22. Vorrichtung nach Anspruch 21, die weiterhin eine Schaltlogik (614) aufweist, die zwischen der Spannungsquelle (612) und der Ladungsplatte (604) angeschlossen ist für das Auswählen von bestimmten Vorstülpungen für die Verbindung mit der Spannungsquelle.

23. Vorrichtung nach Anspruch 22, wobei das Abscheidungsmaterial eine druckbare Substanz ist.

24. Verfahren zum elektrostatischen Abscheiden einer Dosis eines Medikaments, das die Schritte aufweist:
a) Laden einer leitfähigen Oberfläche (416), die in einer beabstandeten Parallelbeziehung zu einer Sammlungsspur (412) angeordnet ist, um eine Spannungsdifferenz zwischen der leitfähigen Oberfläche und der Sammlungsspur zu errichten,
b) Sammeln einer elektrischen Ladung an der Sammlungsspur, wobei die Ladung direkt proportional zu der Spannungsdifferenz ist, die in Schritt a) errichtet wurde,
c) Laden von Medikamentteilchen mit einer Polarität, die entgegengesetzt zu derjenigen der Sammlungsspur ist, und
d) Anordnen der geladenen Medikamententeilchen in der Nähe der Sammlungsspur,
wobei das Medikament elektrostatisch von der Sammlungsspur angezogen wird.

25. Verfahren nach Anspruch 24, das weiterhin den Schritt aufweist des Bestimmens der elektrischen Ladung, in Schritt b) gesammelt wurde.

26. Verfahren nach Anspruch 25, das weiterhin den Schritt aufweist des Unterbrechens des Ladens der leitfähigen Oberfläche, wenn eine bestimmte Menge der elektrischen Ladung auf der Sammlungsspur gesammelt wurde.

27. Verfahren nach Anspruch 26, wobei die Dosis des Medikaments, das auf der Sammlungsspur abgeschieden wird, direkt proportional zu der elektrischen Ladung ist, die auf der Sammlungsspur gesammelt wird.

28. Verfahren nach Anspruch 24, 25, 26 oder 27, wobei die Sammlungsspur einen Ort in einem vorbestimmten Muster definiert, an dem die Medikamententeilchen abzuscheiden sind.

29. Vorrichtung für das elektrostatische Aufnehmen eines Abscheidungsmaterials, wobei die Vorrichtung aufweist:
ein Substrat (104), das eine dielektrische Schicht (108, 410, 600) beinhaltet,
eine leitfähige Platte (106, 416, 617), die auf einer ersten Oberfläche der dielektrischen Schicht angeordnet ist und eine Sammlungsspur (110, 412), die auf einer zweiten Oberfläche der dielektrischen Schicht (108, 410, 600) angeordnet ist, wobei die leitfähige Platte (106, 416, 617) und die Sammlungsspur (110, 412) eine zueinander beabstandete Parallelbeziehung haben, und
eine Spannungsquelle (112, 406, 612), die wahlweise mit der leitfähigen Platte (106, 416, 617) und der Sammlungsspur (110, 412) verbindbar ist, für das Errichten einer Spannungsdifferenz über der dielektrischen Schicht (108) zwischen der Sammlungsspur (110, 412) und der leitfähigen Platte (106, 416, 617) und Laden der Sammlungsspur (110, 412) mit einer ersten Ladungspolarität,
wobei ein Abscheidungsmaterial (102) mit einer zweiten Ladungspolarität, die entgegengesetzt zu der ersten Ladungspolarität ist, elektrostatisch angezogen und auf der Sammlungsspur (110, 412) aufgenommen wird.

30. Vorrichtung nach Anspruch 29, wobei:
das Substrat, das eine dielektrische Schicht (410) beinhalte, eine Mehrzahl von Öffnungen (408) bildet,
die leitfähige Platte (416), die auf einer Oberfläche der dielektrischen Schicht (410) angeordnet ist, Öffnungen definiert, die koaxial in Bezug auf die Öffnungen (408), die von der dielektrischen Schicht (410) gebildet werden, angeordnet sind, und
es eine Mehrzahl von Sammlungsspuren (412) gibt, die auf einer zweiten Oberfläche der dielektrischen Schicht (410) in der Nähe und innerhalb jeder der Öffnungen (408), die von der dielektrischen Schicht (410) gebildet werden, angeordnet sind, und
die Spannungsquelle (406) mit einem ersten Anschluß, der mit der leitfähigen Platte (416) verbunden ist und einem zweiten Anschluß, der mit der Ladungsplatte (404) verbunden ist, für das wahlweise Laden von jeder der Sammlungsspuren (412) mit einer ersten Ladungspolarität vorgesehen ist,
wobei ein Abscheidungsmaterial mit einer zweiten Ladungspolarität, die entgegengesetzt zu der ersten Ladungspolarität ist, elektrostatisch angezogen werden kann und auf den Sammlungsspuren aufgenommen werden kann.

## Revendications

1. Appareil pour la retenue électrostatique d'une matière de dépôt, ledit appareil comprenant :
un substrat (104) incluant une couche diélectrique (108, 410, 600), une plaque conductrice (106, 416, 617), placée sur une première surface de ladite couche diélectrique, et un tracé collecteur (110, 412) placé sur une deuxième surface de ladite couche diélectrique (108, 410, 600), où ladite plaque conductrice (106, 416, 617) et ledit tracé collecteur (110, 412) ont une relation d'espacement parallèle ; dans lequel une différence de potentiel est établie sur la couche diélectrique (108) entre le tracé collecteur (110, 412) et la plaque conductrice (106, 416, 617) ; le tracé collecteur ayant une charge d'une première polarité ; et
une matière de dépôt (102) ayant une deuxième polarité de charge qui est opposée à la première polarité de charge, ledit tracé collecteur (110, 412) attirant et retenant par voie électrostatique ladite matière de dépôt (102).

2. Appareil selon la revendication 1, comprenant de plus une source de tension (112, 406, 612) connectable de manière sélective à ladite plaque conductrice (106, 416, 617) et audit tracé collecteur (110, 412), pour établir ladite différence de potentiel et pour charger ledit tracé collecteur (110, 412) avec la première polarité de charge.

3. Appareil selon la revendication 2, dans lequel ladite source de tension (112, 406, 612) est connectée à ladite plaque conductrice (106, 416, 617) et audit tracé collecteur (110, 412) via un ampèremètre (118, 618).

4. Appareil selon la revendication 2 ou 3, comprenant de plus un moyen de commutation (114; 404; 614, 604, 608, 606) pour la connexion sélective de ladite source de tension (112, 612) audit tracé collecteur (110, 412), pour commander la quantité de charge accumulée sur ledit tracé collecteur (110, 412).

5. Appareil selon la revendication 4, dans lequel ladite couche diélectrique (410) définit une ouverture (408) et ledit tracé collecteur (412) est placé sur ladite première surface à proximité de ladite ouverture et dans ladite ouverture, et dans lequel ladite plaque conductrice (416) définit une ouverture (418) qui est alignée coaxialement avec ladite ouverture (408) définie par ladite couche diélectrique (410).

6. Appareil selon la revendication 5, dans lequel une première borne de ladite source de tension (406) est connectée à ladite plaque conductrice (416) et une deuxième borne de ladite source de tension (406) est connectée à une plaque de charge (404) du moyen de commutation.

7. Appareil selon la revendication 6, dans lequel ladite plaque de charge (404) comprend une protubérance (420) dépassant d'une surface de ladite plaque de charge (404) et étant formée pour interadapter ladite ouverture (418) dans ladite couche diélectrique (410) pour entrer en contact avec ledit tracé collecteur (412) sans entrer en contact avec ladite plaque conductrice (416), pour charger le tracé collecteur.

8. Appareil selon la revendication 7, dans lequel la plaque de charge est mobile entre une première position dans laquelle la protubérance entre en contact avec le tracé collecteur et une deuxième position dans laquelle le tracé n'est pas contacté.

9. Appareil selon l'une des revendications précédentes, dans lequel la matière de dépôt est une poudre.

10. Appareil selon la revendication 9, dans lequel la matière de dépôt est un médicament en poudre.

11. Appareil selon la revendication 10, comprenant de plus un moyen (308), positionné à proximité du tracé collecteur (412), pour extraire ledit médicament en poudre dudit tracé collecteur.

12. Appareil selon la revendication 11, dans lequel ledit moyen d'extraction (308) est un tube de distribution conçu pour l'inhalation dudit médicament en poudre.

13. Appareil selon l'une quelconque des revendications 1 à 8, dans lequel ladite matière de dépôt est un liquide.

14. Appareil selon la revendication 1 dans lequel ledit substrat inclut une couche diélectrique (410) définissant de multiples ouvertures (408) ; ladite plaque conductrice (416) placée sur une première surface de ladite couche diélectrique (410) définit des ouvertures alignées coaxialement aux ouvertures respectives (408) définies par ladite couche diélectrique (410) ; et il y a une multitude de tracés collecteurs (412) placés sur une deuxième surface de ladite couche diélectrique (410) à proximité et dans chacune desdites ouvertures (408) définies par ladite couche diélectrique (410), les tracés collecteurs ayant une charge d'une première polarité et
chacun desdits tracés collecteurs attire et retient par voie électrostatique ladite matière de dépôt et ayant une deuxième polarité de charge qui est opposée à la première polarité de charge.

15. Appareil selon la revendication 14, comprenant de plus une source de tension (406) ayant une première borne connectée à ladite plaque conductrice (416) et une deuxième borne connectée à une plaque de charge (404) pour charger sélectivement chacun desdits tracés collecteurs (412) avec la première polarité de charge.

16. Appareil selon la revendication 15, dans lequel ladite plaque de charge (404) comprend une multitude de protubérances (420) dépassant d'une surface de ladite plaque de charge et étant formées pour interadapter lesdites ouvertures (408) dans ladite couche diélectrique (410) pour entrer en contact avec lesdits tracés collecteurs (412) sans entrer en contact avec ladite plaque conductrice (416).

17. Appareil selon la revendication 15 ou 16, dans lequel la plaque de charge est mobile entre une première position dans laquelle au moins l'une des protubérances entre en contact avec un tracé collecteur et une deuxième position dans laquelle aucun des tracés collecteurs n'est contacté.

18. Appareil selon la revendication 14, 15, 16 ou 17, comprenant de plus
un moyen (306) pour supporter ledit substrat (410, 416, 412) ; et
un moyen (308) pour extraire la matière de dépôt à partir des tracés ;
les tracés (412) pouvant être positionnés par rapport au moyen d'extraction (308) pour permettre au moyen d'extraction (308) d'en extraire la matière.

19. Appareil selon la revendication 14, 15, 16, 17 ou 18, dans lequel la matière de dépôt est un médicament en poudre.

20. Appareil selon la revendication 19, dans lequel ledit moyen d'extraction est un tube de distribution (308) conçu pour l'inhalation dudit médicament en poudre.

21. Appareil selon la revendication 13, 14, 15, 16 ou 17, dans lequel chacun desdits tracés collecteurs (412) remplit chacune desdites ouvertures définies par ladite couche diélectrique (600), les tracés formant un ensemble matriciel de points d'éléments d'impression.

22. Appareil selon la revendication 21, comprenant de plus une logique de commutation (614), connectée entre ladite source de tension (612) et ladite plaque de charge (604), pour sélectionner spécifiquement l'une desdites protubérances pour la connexion à ladite source de tension.

23. Appareil selon la revendication 22, dans lequel ladite matière de dépôt est une substance imprimable.

24. Procédé de dépôt électrostatique d'une dose de médicament comprenant les étapes consistant à :
(a) charger une surface conductrice (416), qui est placée dans une relation d'espacement parallèle avec un tracé collecteur (412), pour établir une différence de potentiel entre la surface conductrice et le tracé collecteur ;
(b) accumuler une charge électrique sur le tracé collecteur, ladite charge étant directement proportionnelle à la différence de potentiel établie à l'étape (a) ;
(c) charger des particules médicamenteuses à une polarité opposée à celle du tracé collecteur ; et
(d) déposer les particules médicamenteuses chargées à proximité du tracé collecteur, le médicament étant ainsi attiré par voie électrostatique au tracé collecteur.

25. Procédé selon la revendication 24, comprenant de plus l'étape de détermination de la charge électrique accumulée à l'étape (b).

26. Méthode selon la revendication 25, comprenant de plus l'étape d'interruption de la charge de la surface conductrice lorsqu'une quantité spécifiée de charge électrique est accumulée au tracé collecteur.

27. Méthode selon la revendication 26, dans laquelle la dose de médicament déposée sur le tracé collecteur est directement proportionnelle à la charge électrique accumulée au tracé collecteur.

28. Méthode selon la revendication 24, 25, 26 ou 27, dans laquelle le tracé collecteur définit un emplacement dans une structure prédéfinie où les particules médicamenteuses doivent être déposées.

29. Appareil pour la retenue électrostatique d'une matière de dépôt, ledit appareil comprenant :
un substrat (104) incluant une couche diélectrique (108, 410, 600),
une plaque conductrice (106, 416, 617), placée sur une première surface de ladite couche diélectrique, et un tracé collecteur (110, 412) placé sur une deuxième surface de ladite couche diélectrique (108, 410, 600), où ladite plaque conductrice (106, 416, 617) et ledit tracé collecteur (110, 412) ont une relation d'espacement parallèle; et
une source de tension (112, 406, 612) connectable de manière sélective à ladite plaque conductrice (106, 416, 617) et audit tracé collecteur (110, 412), pour établir une différence de potentiel sur la couche diélectrique (108) entre le tracé collecteur (110, 412) et la plaque conductrice (106, 416, 617) et pour charger ledit tracé collecteur (110, 412) à une première polarité de charge;
par lequel une matière de dépôt (102), ayant une deuxième polarité de charge qui est opposée à la première polarité de charge, peut être attirée et retenue par voie électrostatique sur ledit tracé collecteur (110, 412).

30. Appareil selon la revendication 20, dans lequel :
ledit substrat incluant une couche diélectrique (410) définit de multiples ouvertures (408);
ladite plaque conductrice (416), placée sur une première surface de ladite couche diélectrique (410), définit des ouvertures alignées coaxialement aux ouvertures respectives (408) définies par ladite couche diélectrique (410); et
il y a une multitude de tracés collecteurs (412), placés sur une deuxième surface de ladite couche diélectrique (410) à proximité et dans chacune desdites ouvertures (408) définies par ladite couche diélectrique (410); et
ladite source de tension (406) ayant une première borne connectée à ladite plaque conductrice (416) et une deuxième borne connectée à une plaque de charge (404), sert à la charge sélective de chacun desdits tracés collecteurs (412) à la première polarité de charge;
par lequel une matière de dépôt, ayant une deuxième polarité de charge qui est opposée à la première polarité de charge, peut être attirée et retenue par voie électrostatique sur lesdits tracés collecteurs.
